(19) Europäisches Patentamt
European Patent Office
Office européen des brevets

(11) Veröffentlichungsnummer: **0 054 866**
**B1**

(12) # EUROPÄISCHE PATENTSCHRIFT

(45) Veröffentlichungstag der Patentschrift: **07.08.85**

(51) Int. Cl.⁴: **C 07 D498/04**, A 61 K 31/505 // (C07D498/04, 239:00, 271:00)

(21) Anmeldenummer: **81110366.2**

(22) Anmeldetag: **11.12.81**

(54) **Neue Oxadiazolopyrimidin-Derivate, Verfahren und Zwischenprodukte zu deren Herstellung sowie Arzneimittel enthaltend ein solches Oxadiazolopyrimidin-Derivat.**

(30) Priorität: **19.12.80 CH 9410/80**
**02.10.81 CH 6350/81**

(43) Veröffentlichungstag der Anmeldung: **30.06.82 Patentblatt 82/26**

(45) Bekanntmachung des Hinweises auf die Patenterteilung: **07.08.85 Patentblatt 85/32**

(84) Benannte Vertragsstaaten: **AT BE CH DE FR GB IT LI LU NL SE**

(56) Entgegenhaltungen:
**DE-A- 2 804 518**
**DE-A- 2 804 519**
**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft**
**CH-4002 Basel (CH)**

(72) Erfinder: **Hengartner, Urs, Dr.**
**Binzenstrasse 28**
**CH-4058 Basel (CH)**
Erfinder: **Muller, Jean-Claude, Dr.**
**2 rue du Collège**
**F-68170 Rixheim (FR)**
Erfinder: **Ramuz, Henri, Dr. Prof.**
**Rheinparkstrasse 3**
**CH-4127 Birsfelden (CH)**

(74) Vertreter: **Lederer, Franz, Dr. et al**
**Patentanwälte Dr. Lederer Franz Meyer-Roxlau Reiner F. Lucile-Grahn-Strasse 22**
**D-8000 München 80 (DE)**

**EP 0 054 866 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Oxadiazolopyrimidinderivate der allgemeinen Formel

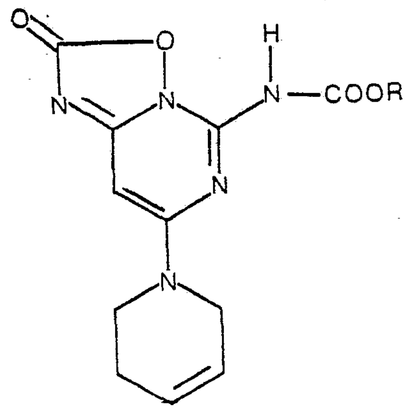

(I)

worin R Alkyl, Alkenyl, Alkinyl oder Alkoxyalkyl bedeutet, sowie Salze davon.

Der in dieser Beschreibung verwendete Ausdruck « Alkyl » — allein oder in Kombination — bezieht sich auf geradkettige und verzweigte, gesättigte Kohlenwasserstoffreste mit 1-8, vorzugsweise 1-4 Kohlenstoffatomen, wie z. B. Methyl, Aethyl, n-Propyl, Isopropyl, n-Butyl, Isobutyl, tert.Butyl und dgl. « Alkoxy » bezieht sich auf Alkyläthergruppen, in denen der Ausdruck « Alkyl » die obige Bedeutung besitzt. Der Ausdruck « Alkenyl » umfasst geradkettige und verzweigte Kohlenwasserstoffreste mit 3-6, vorzugsweise 3 Kohlenstoffatomen, worin mindestens eine Kohlenstoff-Kohlenstoffbindung ungesättigt ist, wie Allyl, Butenyl und dgl. Der Ausdruck « Alkinyl » bezieht sich in ähnlicher Weise auf geradkettige und verzweigte Kohlenwasserstoffreste mit 3-6, vorzugsweise 3 Kohlenstoffatomen, worin mindestens eine Kohlenstoff-Kohlenstoff-Dreifachbindung vorhanden ist, wie Propargyl und dgl.

Von den Verbindungen der Formel I sind diejenigen bevorzugt, in denen R Alkyl oder Alkenyl bedeutet. Besonders bevorzugt sind diejenigen, in denen R Alkyl mit 1-4 Kohlenstoffatomen oder Allyl bedeutet. Ganz besonders bevorzugt sind diejenigen Verbindungen der Formel I, worin R Butyl oder Allyl bedeutet, d. h. Butyl 7-[3,6-dihydro-1(2H)-pyridyl]-2-oxo-2H-[1,2,4]-oxadiazolo [2,3-c] pyrimidin-5-carbamat und Allyl 7-[3,6-dihydro-1(2H)-pyridyl]-2-oxo-2H-[1,2,4] oxadiazolo [2,3-c]-pyrimidin-5-carbamat.

Aus den beiden Deutschen Offenlegungsschriften 2.804.518 und 2.804.519 sind Oxadiazolo [2,3-a] pyrimidin-Derivate bekannt, die sich demnach nur durch die Verknüpfungsstelle der beiden Heterocyclen von den erfindungsgemässen Oxadiazolo [2,3-c] pyrimidin-Derivaten unterscheiden. Die vorbekannten Verbindungen besitzen ebenfalls gefässerweiternde und/oder blutdrucksenkende Eigenschaften.

Die Verbindungen der Formel I sowie deren Salze können erfindungsgemäss so hergestellt werden, dass man

a) 5-Amino-7-[3,6-dihydro-1(2H)-pyridyl]-2H-[1,2,4] oxadiazolo [2,3-c] pyrimidin-2-on der Formel

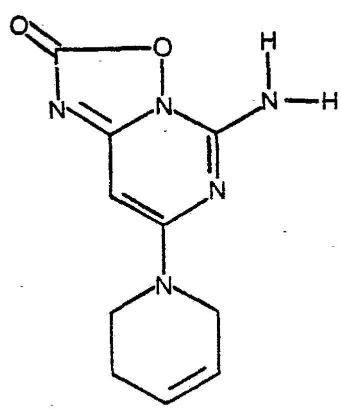

(II)

mit einem Azolderivat der allgemeinen Formel

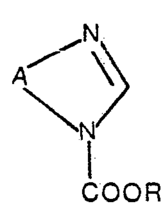

(III)

worin R die obige Bedeutung besitzt und A eine der Gruppen —CH=CH—,

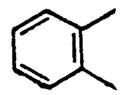

oder —N=CH— bedeutet, wobei sich das Stickstoffatom im letzteren Falle in 2-Stellung befindet, umsetzt, und

b) erwünschtenfalls eine erhaltene Verbindung der Formel I in ein Salz oder ein Salz in ein anderes Salz überführt.

Die Umsetzung der Verbindung der Formel II mit einem Azolderivat der Formel III erfolgt nach an sich bekannten Methoden. Zweckmässig wird das Anion der Verbindung der Formel II mit dem Azolderivat der Formel III zur Reaktion gebracht. Das Anion der Verbindung der Formel II wird vorteilhafterweise in situ durch Umsetzen mit einer Base hergestellt. Für diesen Zweck geeignet sind Basen, wie Alkalimetallhydride, z. B. Natriumhydrid, Alkalimetallamide, z. B. Natrium- oder Kaliumamid, Lithiumdiisopropylamid, Kalium-t.-butylat, und dgl. Die Umsetzung erfolgt in einem unter den Reaktionsbedingungen inerten Lösungsmittel bei einer Temperatur zwischen etwa – 25 °C und Raumtemperatur, vorzugsweise zwischen etwa 0° und 15 °C. Als Lösungsmittel kommen Dimethylformamid, ein gesättigter Kohlenwasserstoff, z. B. Hexan, ein aromatischer Kohlenwasserstoff, z. B. Benzol, Toluol oder Xylol, ein Aether, z. B. Diäthyläther, Dioxan oder Tetrahydrofuran, und dgl. in Frage.

Der Ausgangsstoff der Formel II ist neu und ebenfalls Gegenstand der vorliegenden Erfindung. Er kann beispielsweise durch Cyclisation einer Verbindung der allgemeinen Formel

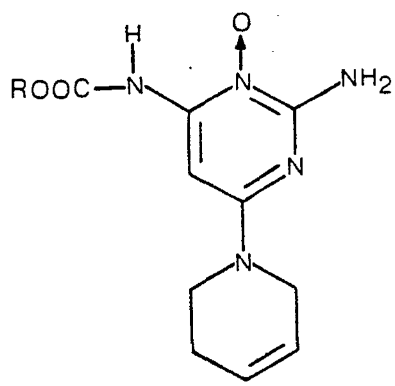

(IV)

worin R die obige Bedeutung besitzt, hergestellt werden.

Die Cyclisation einer Verbindung der Formel IV erfolgt in an sich bekannter Weise durch Erhitzen auf eine Temperatur zwischen etwa 50° und 200 °C, vorzugsweise zwischen etwa 100° und 150 °C. Die Reaktion kann in Abwesenheit oder Gegenwart eines Lösungsmittels oder Lösungsmittelgemisches durchgeführt werden. Wird die Reaktion in einem Lösungsmittel bzw. Lösungsmittelgemisch durchgeführt, so kommen als Lösungsmittel aromatische Kohlenwasserstoffe wie Benzol, Toluol oder Xylol, Aether wie Dibutyläther, Dioxan oder Diäthylenglykoldimethyläther, Dimethylformamid und dgl. oder Gemische davon in Frage. Es ist klar, dass man entweder ein Lösungsmittel verwenden kann, dessen Siedepunkt höher liegt als die Reaktionstemperatur, oder dass man ein im weiter oben angegebenen Temperaturbereich siedendes Lösungsmittel bei dessen Rückflusstemperatur verwenden kann. Vorzugsweise wird die Reaktion unter Verwendung von Dimethylformamid oder Toluol als Lösungsmittel durchgeführt. Die Reaktionszeit hängt von der verwendeten Reaktionstemperatur ab und liegt zwischen etwa 1/4 und 18 Stunden. Arbeitet man im bevorzugten Temperaturbereich zwischen etwa 100° und 150 °C, so beträgt die Reaktionszeit etwa 1/4 bis 12 Stunden, vorzugsweise 1/4 bis 2 Stunde.

Die Verbindungen der Formeln III und IV gehören bekannten Verbindungsklassen an. Sie sind demnach entweder spezifisch vorbeschrieben oder können in Analogie zur Herstellung der vorbekannten Verbindungen erhalten werden.

Die Verbindungen der Formel I können in Salze übergeführt werden, beispielsweise durch Behandlung mit einer anorganischen Base, wie einem Alkalihydroxyd, beispielsweise Natriumhydroxyd oder Kaliumhydroxyd, oder einem Erdalkalihydroxyd, beispielsweise Calciumhydroxyd. Salze der Verbindungen der Formel I können auch durch Umsalzen eines geeigneten Salzes hergestellt werden. Von den Salzen der Verbindungen der Formel I sind die pharmazeutisch verwendbaren bevorzugt.

Die Verbindungen der Formel I sowie deren Salze besitzen langanhaltende wertvolle gefässerweiternde und/oder blutdrucksenkende Eigenschaften und können somit zur Behandlung gefässbedingter Hypertonien oder auch als Vasodilatatoren bei peripheren Durchblutungsstörungen Anwendung finden.

Die blutdrucksenkende Wirkung kann nach folgender Methode an wachen, spontan hypertonen Ratten bestimmt werden :

Der systolische Blutdruck und die Herzfrequenz werden von Substanzgabe zweimal gemessen. Die

Substanzgabe erfolgt mittels Schlundsonde zweimal täglich, morgens und nachmittags. Beide Parameter werden 1, 3, 6 und 24 Stunden nach der Applikation gemessen und die prozentualen Veränderungen zu den Kontrollwerten berechnet. Der systolische Blutdruck wird indirekt an der Schwanzarterie der Ratte nach der Methode von Gerold et al. gemessen (Helv. Physiol. Acta *24* : 58-69, 1966 ; Arzneimittelforschung *18* : 1285-1287, 1968).

In der nachfolgenden Tabelle sind die erhaltenen Resultate zusammengestellt, wobei jeweils die maximalen prozentualen Abweichungen von den Kontrollwerten angegeben sind. Daneben sind in der nachfolgenden Tabelle auch die Toxizitätswerte aufgeführt.

Tabelle

| Verbindung | Dosis $mg.kg^{-1}$ | Blut-druck Δ% | Herz-frequenz Δ% | Tox. $DL_{50}$ $mg.kg.^{-1}$ p.o. |
|---|---|---|---|---|
| A | 30 | -14,2 | +10,0 | >4'000 |
| | 100 | -38,4 | +13,0 | |
| B | 1 | -12,6 | + 8,2 | >5'000 |
| | 3 | -33,4 | +20,0 | |
| | 10 | -40,0 | +21,2 | |
| C | 10 | -21,8 | +12,7 | >4'000 |
| | 30 | -27,0 | +14,3 | |
| D | 10 | -31,0 | + 9,0 | >5'000 |
| E | 1 | -24,1 | +12,5 | >5'000 |
| | 3 | -40,9 | + 9,4 | |
| F | 10 | -37,3 | +12,7 | >5'000 |
| G | 30 | -27,9 | +12,2 | >5'000 |
| H | 0,3 | -11,3 | +17,7 | >4'000 |
| | 1 | -19,8 | +12,8 | |
| | 3 | -31,6 | -10,1 | |
| | 10 | -51,3 | - 9,1 | |

A = Methyl 7-[3,6-dihydro-1(2H)-pyridyl]-2-oxo-2H-[1,2,4] oxadiazolo [2,3-c] pyrimidin-5-carbamat
B = Isobutyl 7-[3,6-dihydro-1(2H)-pyridyl]-2-oxo-2H-[1,2,4] oxadiazolo [2,3-c] pyrimidin-5-carbamat
C = (2-Methoxyäthyl) 7-[3,6-dihydro-1(2H)-pyridyl]-2-oxo-2H-[1,2,4] oxadiazolo [2,3-c] pyrimidin-5-carbamat
D = Butyl 7-[3,6-dihydro-1(2H)-pyridyl]-2-oxo-2H-[1,2,4] oxadiazolo [2,3-c] pyrimidin-5-carbamat
E = Propyl 7-[3,6-dihydro-1(2H)-pyridyl]-2-oxo-2H-[1,2,4] oxadiazolo [2,3-c] pyrimidin-5-carbamat
F = Isopropyl 7-[3,6-dihydro-1(2H)-pyridyl]-2-oxo-2H-[1,2,4] oxadiazolo [2,3-c] pyrimidin-5-carbamat
G = Propargyl 7-[3,6-dihydro-1(2H)-pyridyl]-2-oxo-2H-[1,2,4] oxadiazolo [2,3-c] pyrimidin-5-carbamat
H = Allyl 7-[3,6-dihydro-1(2H)-pyridyl]-2-oxo-2H-[1,2,4] oxadiazolo [2,3-c] pyrimidin-5-carbamat

Die Verfahrensprodukte und deren pharmazeutisch verwendbare Salze können als Heilmittel z. B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z. B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z. B. als Tabletten, Dragées, Suppositorien, Kapseln oder in flüssiger Form, z. B. als Lösungen, Suspensionen oder Emulsionen, vorliegen. Gegebenenfalls sind sie

sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle Stoffe enthalten.

Die tägliche Dosis bei oraler Verabreichung liegt zwischen etwa 10 und 500 mg ; i. v.-Verabreichung zwischen etwa 1 und 50 mg. Die angegebenen Dosierungen sind jedoch nur als Beispiele zu verstehen und können je nach der Schwere des zu behandelnden Falles und nach Ermessen des behandelnden Arztes abgeändert werden.

Die nachfolgenden Beispiele erläutern die Erfindung. Alle Temperaturen sind in Celsiusgraden angegeben. Die Schmelzpunkte sind nicht korrigiert.

## Beispiel 1

4,30 g (98-108 mMol) Natriumhydrid (55-60 %ige Dispersion in Oel) werden mit Hexan gewaschen und unter Argon in 150 ml Dimethylformamid suspendiert. 20,54 g (88 mMol) 5-Amino-7-[3,6-dihydro-1(2H)-pyridyl]-2H-[1,2,4]-oxadiazolo [2,3-c] pyrimidin-2-on werden portionenweise innerhalb von 10 Minuten in die gerührte, eisgekühlte Suspension eingetragen, wobei die Temperatur bei 14 °C gehalten wird. Das Eisbad wird entfernt und das Reaktionsgemisch während 30 Minuten gerührt. Dann wird eine Lösung von 15,8 g (125 mMol) 1-Methoxycarbonyl-imidazol in 50 ml Dimethylformamid rasch zugegeben und die Reaktionslösung bei 20-25° während 1 Stunde gerührt. Die klare, rotbraune Lösung wird in 1,2 l Wasser gegossen, die leicht trübe Lösung durch ein Speedex-Filterbett filtriert und unter Rühren mit 40 ml Eisessig versetzt. Der Niederschlag wird abfiltriert und mit 600 ml Wasser gründlich gewaschen. Der Filterkuchen wird in der Nutsche mit 400 ml Methanol aufgeschlämmt und scharf abgesaugt. Nach dem Trocknen (40°, 20 mm Hg) erhält man 23,37 g (91 %) Methyl 7-[3,6-dihydro-1(2H)-pyridyl]-2-oxo-2H-[1,2,4] oxadiazolo [2,3-c] pyrimidin-5-carbamat als weisses Pulver, Smp. 213° (Zersetzung).

10,00 g des Rohprodukts werden in 500 ml Methanol und 25 ml Triäthylamin unter Rückfluss gelöst und filtriert. Die warme Lösung wird innerhalb von 30 Minuten mit 20 ml Eisessig in 50 ml Methanol versetzt ; die Suspension wird für weitere 30 Minuten bei Raumtemperatur gerührt. Das Kristallisat wird abfiltriert und mit 3 × 200 ml Methanol gründlich gewaschen. Nach dem Trocknen (40°, 20 mm Hg) erhält man 8,99 g weisse Kristalle, Smp. 215° (Zersetzung).

Das als Ausgangsmaterial eingesetzte 5-Amino-7-[3,6-dihydro-1(2H)-pyridyl]-2H-[1,2,4] oxadiazolo [2,3-c] pyrimidin-2-on kann wie folgt hergestellt werden :

28,8 g (0,109 Mol) Methyl 2-amino-6-[3,6-dihydro-1(2H)-pyridyl]-4-pyrimidincarbamat-3-oxid werden mit 350 ml Dimethylformamid versetzt und in einem Oelbad von 135° während 30 Minuten gerührt. Die braune Reaktionslösung wird unter vermindertem Druck eingedampft. Der noch feuchte Rückstand wird für 1 Stunde in 350 ml Aether suspendiert, das Kristallisat abfiltriert und mit Aether gewaschen. Nach dem Trocknen (40°, 20 mm Hg) erhält man 23,4 g (92,5 %) leicht beiges, kristallines Pulver, Smp. 228° (Zersetzung).

Das als Ausgangsmaterial eingesetzte 1-Methoxycarbonylimidazol kann wie folgt hergestellt werden :

Zu einer gerührten Lösung von 34,05 g (0,50 Mol) Imidazol und 52,5 g (0,52 Mol) Triäthylamin in 500 ml Acetonitril werden bei 10° innerhalb von 40 Minuten 47,2 g (0,50 Mol) Chlorameisensäuremethylester in 60 ml Aether getropft. Die Suspension wird während 30 Minuten bei Raumtemperatur gerührt. Der Niederschlag wird abfiltriert und mit Aether gewaschen. Das Filtrat wird unter vermindertem Druck eingedampft, der Rückstand in 500 ml Benzol aufgenommen und 10 Minuten gerührt. Das unlösliche Material wird wiederum abfiltriert und das Filtrat unter vermindertem Druck eingedampft, wobei das verbleibende Oel langsam erstarrt. Man erhält 61,7 g (98 %) eines leicht gelblichen Festkörpers, Smp. 35-40°, der ohne weitere Reinigung eingesetzt werden kann.

## Beispiel 2

In zu Beispiel 1 analoger Weise erhält man durch Umsetzen von 5-Amino-7-[3,6-dihydro-1(2H)-pyridyl]-2H-[1,2,4] oxadiazolo [2,3-c] pyrimidin-2-on mit

1-Isobutyloxycarbonyl-imidazol das Isobutyl 7-[3,6-dihydro-1(2H)-pyridyl]-2-oxo-2H-[1,2,4] oxadiazolo [2,3-c] pyrimidin-5-carbamat, Smp. 197-198° (aus Methylenchlorid/Methanol/Diäthyläther),

1-(2-Methoxyäthyl)oxycarbonyl-imidazol das (2-Methoxyäthyl) 7-[3,6-dihydro-1(2H)-pyridyl]-2-oxo-2H-[1,2,4]-oxadiazolo [2,3-c] pyrimidin-5-carbamat, Smp. 203-204° (aus Methylenchlorid/Methanol/Diäthyläther),

1-Butyloxycarbonyl-imidazol das Butyl 7-[3,6-dihydro-1(2H)-pyridyl]-2-oxo-2H-[1,2,4] oxadiazolo [2,3-c] pyrimidin-5-carbamat, Smp. 197-198° (aus Methylenchlorid/Methanol/Diäthyläther),

1-Aethoxycarbonyl-imidazol das Aethyl 7-[3,6-dihydro-1(2H)-pyridyl]-2-oxo-2H-[1,2,4] oxadiazolo [2,3-c] pyrimidin-5-carbamat, Smp. 202-204° (aus Methylenchlorid/Methanol/Diäthyläther),

1-Propyloxycarbonyl-imidazol das Propyl 7-[3,6-dihydro-1(2H)-pyridyl]-2-oxo-2H-[1,2,4] oxadiazolo [2,3-c] pyrimidin-5-carbamat, Smp. 201-202° (aus Methylenchlorid/Methanol/Diäthyläther),

1-Isopropyloxycarbonyl-imidazol das Isopropyl 7-[3,6-dihydro-1(2H)-pyridyl]-2-oxo-2H-[1,2,4] oxadiazolo [2,3-c] pyrimidin-5-carbamat, Smp. 186-187° (aus Methylenchlorid/Methanol/Diäthyläther),

1-Propargyloxycarbonyl-imidazol das Propargyl 7-[3,6-dihydro-1(2H)-pyridyl]-2-oxo-2H-[1,2,4] oxadiazolo [2,3-c] pyrimidin-5-carbamat, Smp. 211-213° (aus Methylenchlorid/Methanol/Diäthyläther) und
1-Allyloxycarbonyl-imidazol das Allyl 7-[3,6-dihydro-1(2H)-pyridyl]-2-oxo-2H-[1,2,4] oxadiazolo [2,3-c] pyrimidin-5-carbamat, Smp. 207-209° (aus Methylenchlorid/Methanol/Diäthyläther).

Die als Ausgangsstoffe eingesetzten, oben genannten Imidazolderivate werden in zu Beispiel 1, letzter Absatz analoger Weise durch Umsetzen von Imidazol mit dem entsprechenden Chlorameisensäureester hergestellt. Da diese Derivate bei Raumtemperatur nicht kristallisieren, werden sie mit Hilfe spektroskopischer Methoden charakterisiert.

## Beispiel 3

Zu einer auf 15° gekühlten Lösung von 4,66 g (20 mMol) 5-Amino-7-[3,6-dihydro-1(2H)-pyridyl]-2H-[1,2,4] oxadiazolo [2,3-c] pyrimidin-2-on in 200 ml Dimethylformamid gibt man 1,2 g Natriumhydrid portionenweise zu. Das Gemisch wird während 30 Minuten bei Raumtemperatur stark gerührt. Danach gibt man 5,28 g 1-Methoxycarbonyl-benzimidazol in 50 ml Dimethylformamid zu. Nach 2 Stunden wird das Reaktionsgemisch mit eiskaltem Wasser versetzt und mit Eisessig auf pH 5 eingestellt. Der dabei entstehende Niederschlag wird aus Methylenchlorid/Methanol umkristallisiert, wobei man Methyl 7-[3,6-dihydro-1(2H)-pyridyl]-2-oxo-2H-[1,2,4] oxadiazolo [2,3-c] pyrimidin-5-carbamat erhält, Smp. 212-214°.

Das als Ausgangsmaterial eingesetzte 1-Methoxycarbonylbenzimidazol kann wie folgt hergestellt werden :

Zu einer Lösung von 11,8 g (100 mMol) Benzimidazol in Acetonitril werden 16, 72 ml Triäthylamin zugetropft. Dazu gibt man bei 10° eine Lösung von 7,68 ml Chlorameisensäuremethylester in 30 ml Aether. Der Niederschlag wird abfiltriert, und die Lösung unter vermindertem Druck eingedampft. Das so erhaltene 1-Methoxycarbonyl-benzimidazol wird ohne weitere Reinigung in die nächste Stufe eingesetzt.

## Beispiel 4

13 g Methyl 7-[3,6-dihydro-1(2H)-pyridyl]-2-oxo-2H-[1,2,4] oxadiazolo [2,3-c] pyrimidin-5-carbamat werden in 100 ml Wasser gerührt und langsam mit 44,7 ml 1N wässriger Natronlauge (1,001 Moläquivalente) versetzt, wobei die pH-Aenderung bis zu deren Stabilisierung verfolgt wird. Sobald der pH-Wert stabil ist, wird die erhaltene Lösung filtriert, gefriergetrocknet und lyophilisiert. Nach nochmaligem Trocknen bei 50° im Hochvakuum erhält man 13,5 g des Natriumsalzes von Methyl 7-[3,6-dihydro-1(2H)-pyridyl]-2-oxo-2H-[1,2,4] oxadiazolo [2,3-c] pyrimidin-5-carbamat in Form eines schönen weissen Pulvers vom Schmelzpunkt 192-198°.

In analoger Weise erhält man aus 33 g Butyl 7-[3,6-dihydro-1(2H)-pyridyl]-2-oxo-2H-[1,2,4] oxadiazolo [2,3-c] pyrimidin-5-carbamat und 1,001 Moläquivalenten 1N wässriger Natronlauge das entsprechende Natriumsalz vom Schmelzpunkt 158-169°.

## Beispiel A

Herstellung von Tabletten folgender Zusammensetzung :

| | | |
|---|---|---|
| I | Butyl 7-[3,6-dihydro-1(2H)-pyridyl]-2-oxo-2H-[1,2,4] oxadiazolo [2,3-c] pyrimidin-5-carbamat mikronisiert | 20,0 mg |
| | Milchzucker pulv. | 40,0 mg |
| | Maisstärke weiss | 24,9 mg |
| II | Dioctylnatriumsulfosuccinat | 0,1 mg |
| | Maisstärke weiss | 5,0 mg |
| | Wasser | q.s. |
| III | Maisstärke weiss | 6,0 mg |
| IV | Talk | 3,0 mg |
| | Magnesiumstearat | 1,0 mg |
| | | 100,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die pressfertige Mischung wird zu Tabletten à 100 mg von 7 mm Durchmesser mit Bruchrille verpresst.

## Beispiel B

Herstellung von Tabletten folgender Zusammensetzung :

| | | |
|---|---|---|
| I | Butyl 7-[3,6-dihydro-1(2H)-pyridyl]-2-oxo-2H-[1,2,4] oxadiazolo [2,3-c] pyrimidin-5-carbamat mikronisiert | 200,0 mg |
| | Milchzucker pulv. | 42,9 mg |
| | Maisstärke weiss | 50,0 mg |
| II | Dioctylnatriumsulfosuccinat | 0,1 mg |
| | Maisstärke weiss | 20,0 mg |
| | Wasser | q.s. |
| III | Maisstärke weiss | 30,0 mg |
| IV | Talk | 3,5 mg |
| | Magnesiumstearat | 3,5 mg |
| | | 350,0 mg |

Die Stoffe der Phase I werden gesiebt und gemischt. Diese Mischung wird mit dem Maisstärkekleister II bedeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die pressfertige Mischung wird zu Tabletten à 350 mg von 11 mm Durchmesser mit Bruchrille verpresst.

## Beispiel C

Herstellung von Kapseln folgender Zusammensetzung :

| | | |
|---|---|---|
| I | Butyl 7-[3,6-dihydro-1(2H)-pyridyl]-2-oxo-2H-[1,2,4] oxadiazolo [2,3-c] pyrimidin-5-carbamat mikronisiert | 20,0 mg |
| | Milchzucker pulv. | 48,0 mg |
| II | Maisstärke | 5,0 mg |
| | Wasser | q.s. |
| III | Milchzucker krist. | 50,0 mg |
| | Maisstärke | 15,0 mg |
| IV | Talk | 10,0 mg |
| | Magnesiumstearat | 2,0 mg |
| | | 150,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die Kapselmischung wird zu je 150 mg in Kapseln Grösse 2 gefüllt.

## Beispiel D

Man stellt eine wässrige Tropfensuspension folgender Zusammensetzung her :

| | 10 mg pro 1 ml |
|---|---|
| Butyl 7-[3,6-dihydro-1(2H)-pyridyl]-2-oxo-2H-[1,2,4] oxadiazolo [2,3-c]-pyrimidin-5-carbamat mikronisiert | 0,1 g |
| Natriumbenzoat | 0,035 g |
| Saccharin-Natrium | 0,015 g |
| Acrylsäurepolymerisat | 0,1-1,0 g |
| Saccharose | 3,5 g |
| Citronensäure | 0,025 g |
| Polyoxyäthylen-stearat | 0,002-0,1 g |
| Natriumhydroxid | q.s. |

| | |
|---|---|
| Aroma | q.s. |
| Lebensmittelfarbstoff | q.s. |
| Wasser entsalzt | ad 10,0 ml |

Beispiel E

Wenn man nach den in den Beispielen A-D beschriebenen Verfahren arbeitet, können aus den folgenden, ebenfalls bevorzugten Verbindungen und ihrer pharmazeutisch verwendbaren Salzen Tabletten, Kapseln und Injektionspräparate hergestellt werden :

Methyl 7-[3,6-dihydro-1(2H)-pyridyl]-2-oxo-2H-[1,2,4] oxadiazolo [2,3-c] pyrimidin-5-carbamat,
Aethyl 7-[3,6-dihydro-1(2H)-pyridyl]-2-oxo-2H-[1,2,4] oxadiazolo [2,3-c] pyrimidin-5-carbamat,
Propyl 7-[3,6-dihydro-1(2H)-pyridyl]-2-oxo-2H-[1,2,4] oxadiazolo [2,3-c] pyrimidin-5-carbamat,
Isopropyl 7-[3,6-dihydro-1(2H)-pyridyl]-2-oxo-2H-[1,2,4] oxadiazolo [2,3-c] pyrimidin-5-carbamat,
Isobutyl 7-[3,6-dihydro-1(2H)-pyridyl]-2-oxo-2H-[1,2,4] oxadiazolo [2,3-c] pyrimidin-5-carbamat und
Allyl 7-[3,6-dihydro-1(2H)-pyridyl]-2-oxo-2H-[1,2,4] oxadiazolo [2,3-c] pyrimidin-5-carbamat.

**Patentansprüche** (für die Vertragsstaaten : BE, CH, DE, FR, GB, IT, LI, LU, NL, SE)

1. Oxadiazolopyrimidinderivate der allgemeinen Formel

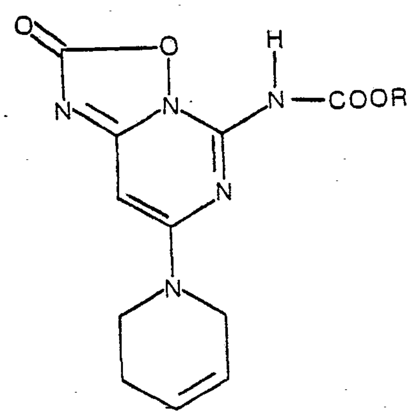

(I)

worin R $C_1$-$C_8$-Alkyl, $C_3$-$C_6$-Alkenyl, $C_3$-$C_6$-Alkinyl oder $C_2$-$C_8$-Alkoxyalkyl bedeutet, sowie Salze davon.

2. Verbindungen nach Anspruch 1, worin R $C_1$-$C_8$-Alkyl oder $C_2$-$C_8$-Alkoxyalkyl bedeutet.
3. Verbindungen nach Anspruch 1, worin R $C_1$-$C_8$-Alkyl oder $C_3$-$C_6$-Alkenyl bedeutet.
4. Verbindungen nach Anspruch 3, worin R Alkyl mit 1-4 Kohlenstoffatomen oder Allyl bedeutet.
5. Methyl 7-[3,6-dihydro-1(2H)-pyridyl]-2-oxo-2H-[1,2,4] oxadiazolo [2,3-c] pyrimidin-5-carbamat.
6. Aethyl 7-[3,6-dihydro-1(2H)-pyridyl]-2-oxo-2H-[1,2,4] oxadiazolo [2,3-c] pyrimidin-5-carbamat.
7. Isobutyl 7-[3,6-dihydro-1(2H)-pyridyl]-2-oxo-2H-[1,2,4] oxadiazolo [2,3-c] pyrimidin-5-carbamat.
8. Butyl 7-[3,6-dihydro-1(2H)-pyridyl]-2-oxo-2H-[1,2,4] oxadiazolo [2,3-c] pyrimidin-5-carbamat.
9. Propyl 7-[3,6-dihydro-1(2H)-pyridyl]-2-oxo-2H-[1,2,4] oxadiazolo [2,3-c] pyrimidin-5-carbamat.
10. Isopropyl 7-[3,6-dihydro-1(2H)-pyridyl]-2-oxo-2H-[1,2,4] oxadiazolo [2,3-c] pyrimidin-5-carbamat.
11. Allyl 7-[3,6-dihydro-1(2H)-pyridyl]-2-oxo-2H-[1,2,4] oxadiazolo [2,3-c] pyrimidin-5-carbamat.
12. Salze der Verbindungen gemäss Ansprüche 5-11.
13. 5-Amino-7-[3,6-dihydro-1(2H)-pyridyl]-2H-[1,2,4] oxadiazolo [2,3-c] pyrimidin-2-on der Formel

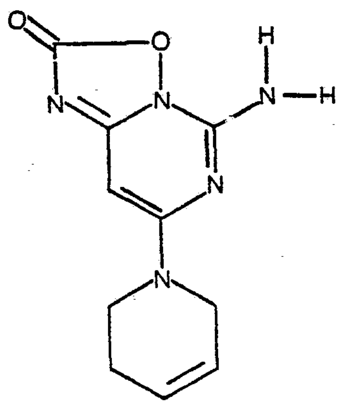

(II)

14. Oxadiazolopyrimidin-Derivate gemäss einem der Ansprüche 1-11 und deren pharmazeutisch verwendbare Salze als pharmazeutische Wirkstoffe.

15. Oxadiazolopyrimidin-Derivate gemäss einem der Ansprüche 1-11 und deren pharmazeutisch verwendbare Salze als Antihypertensiva oder Vasodilatatoren.

16. Verfahren zur Herstellung von Oxadiazolopyrimidin-Derivaten der in Anspruch 1 definierten allgemeinen Formel I und ihren Salze, dadurch gekennzeichnet, dass man

a) 5-Amino-7-[3,6-dihydro-1(2H)-pyridyl]-2H-[1,2,4] oxadiazolo [2,3-c] pyrimidin-2-on der Formel

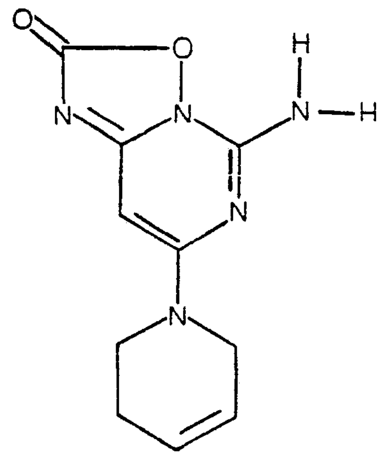

(II)

mit einem Azolderivat der allgemeinen Formel

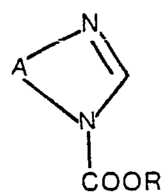

(III)

worin R die in Anspruch 1 angegebene Bedeutung besitzt und A eine der Gruppen —CH=CH—,

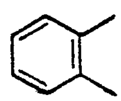

oder —N=CH— bedeutet, wobei sich das Stickstoffatom im letzteren Falle in 2-Stellung befindet, umsetzt, und

b) erwünschtenfalls eine erhaltene Verbindung der Formel I in ein Salz oder ein Salz in ein anderes Salz überführt.

17. Arzneimittel enthaltend ein Oxadiazolopyrimidin-Derivat gemäss einem der Ansprüche 1-11 oder ein pharmazeutisch verwendbares Salz davon.

18. Antihypertensivum oder Vasodilatator enthaltend ein Oxadiazolopyrimidin-Derivat gemäss einem der Ansprüche 1-11 oder ein pharmazeutisch verwendbares Salz davon.

**Patentansprüche** (für den Vertragsstaat AT)

1. Verfahren zur Herstellung von Oxadiazolopyrimidinderivaten der allgemeinen Formel

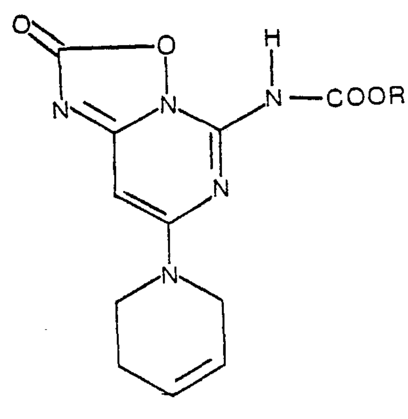

(I)

worin R $C_1$-$C_8$-Alkyl, $C_3$-$C_6$-Alkenyl, $C_3$-$C_6$-Alkinyl oder $C_2$-$C_8$-Alkoxyalkyl bedeutet, und ihren Salzen, dadurch gekennzeichnet, dass man

a) 5-Amino-7-[3,6-dihydro-1(2H)-pyridyl]-2H-[1,2,4] oxadiazolo [2,3-c] pyrimidin-2-on der Formel

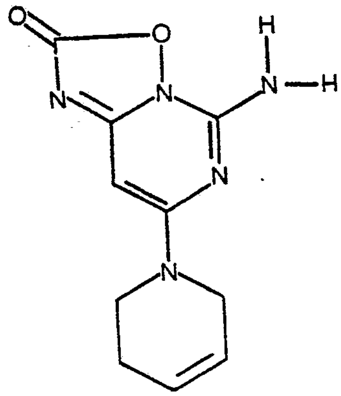

(II)

mit einem Azolderivat der allgemeinen Formel

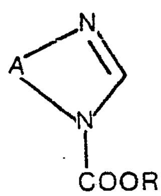

(III)

worin R die obige Bedeutung besitzt und A eine der Gruppen —CH=CH—,

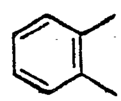

oder —N=CH— bedeutet, wobei sich das Stickstoffatom im letzteren Falle in 2-Stellung befindet, umsetzt, und

b) erwünschtenfalls eine erhaltene Verbindung der Formel I in ein Salz oder ein Salz in ein anderes Salz überführt.

2. Verfahren nach Anspruch 1, dadurch gekennzeichnet, dass R $C_1$-$C_8$-Alkyl oder $C_2$-$C_8$-Alkoxyalkyl bedeutet.

3. Verfahren nach Anspruch 1, dadurch gekennzeichnet, dass R $C_1$-$C_8$-Alkyl oder $C_3$-$C_6$-Alkenyl bedeutet.

4. Verfahren nach Anspruch 3, dadurch gekennzeichnet, dass R Alkyl mit 1-4 Kohlenstoffatomen oder Allyl bedeutet.

5. Verfahren nach Anspruch 2, dadurch gekennzeichnet, dass man Methyl 7-[3,6-dihydro-1(2H)-pyridyl]-2-oxo-2H-[1,2,4] oxadiazolo [2,3-c] pyrimidin-5-carbamat herstellt.

6. Verfahren nach Anspruch 2, dadurch gekennzeichnet, dass man Aethyl 7-[3,6-dihydro-1(2H)-pyridyl]-2-oxo-2H-[1,2,4] oxadiazolo [2,3-c] pyrimidin-5-carbamat herstellt.

7. Verfahren nach Anspruch 2, dadurch gekennzeichnet, dass man Isobutyl 7-[3,6-dihydro-1(2H)-pyridyl]-2-oxo-2H-[1,2,4] oxadiazolo [2,3-c] pyrimidin-5-carbamat herstellt.

8. Verfahren nach Anspruch 2, dadurch gekennzeichnet, dass man Butyl 7-[3,6-dihydro-1(2H)-pyridyl]-2-oxo-2H-[1,2,4] oxadiazolo [2,3-c] pyrimidin-5-carbamat herstellt.

9. Verfahren nach Anspruch 4, dadurch gekennzeichnet, dass man Propyl 7-[3,6-dihydro-1(2H)-pyridyl]-2-oxo-2H-[1,2,4] oxadiazolo [2,3-c] pyrimidin-5-carbamat herstellt.

10. Verfahren nach Anspruch 4, dadurch gekennzeichnet, dass man Isopropyl 7-[3,6-dihydro-1(2H)-pyridyl]-2-oxo-2H-[1,2,4] oxadiazolo [2,3-c] pyrimidin-5-carbamat herstellt.

11. Verfahren nach Anspruch 4, dadurch gekennzeichnet, dass man Allyl 7-[3,6-dihydro-1(2H)-pyridyl]-2-oxo-2H-[1,2,4] oxadiazolo [2,3-c] pyrimidin-5-carbamat herstellt.

12. Verfahren nach Anspruch 1, dadurch gekennzeichnet, dass man ein Salz einer der Verbindungen gemäss einem der Ansprüche 5-11 herstellt.

**Claims** (for the Contracting States : BE, CH, DE, FR, GB, IT, LI, LU, NL, SE)

1. Oxadiazolopyrimidine derivatives of the general formula

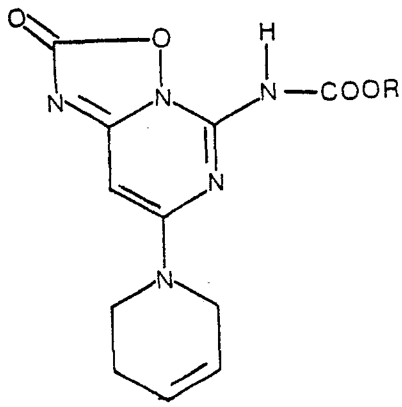

(I)

wherein R signifies $C_1$-$C_8$-alkyl, $C_3$-$C_6$-alkenyl, $C_3$-$C_6$-alkynyl or $C_2$-$C_8$-alkoxyalkyl, as well as salts thereof.

2. Compounds according to claim 1, wherein R signifies $C_1$-$C_8$-alkyl or $C_2$-$C_8$-alkoxyalkyl.
3. Compounds according to claim 1, wherein R signifies $C_1$-$C_8$-alkyl or $C_3$-$C_6$-alkenyl.
4. Compounds according to claim 3, wherein R signifies alkyl with 1-4 carbon atoms or allyl.
5. Methyl 7-[3,6-dihydro-1(2H)-pyridyl]-2-oxo-2H-[1,2,4] oxadiazolo [2,3-c] pyrimidine-5-carbamate.
6. Ethyl 7-[3,6-dihydro-1(2H)-pyridyl]-2-oxo-2H-[1,2,4] oxadiazolo [2,3-c] pyrimidine-5-carbamate.
7. Isobutyl 7-[3,6-dihydro-1(2H)-pyridyl]-2-oxo-2H-[1,2,4] oxadiazolo [2,3-c] pyrimidine-5-carbamate.
8. Butyl 7-[3,6-dihydro-1(2H)-pyridyl]-2-oxo-2H-[1,2,4] oxadiazolo [2,3-c] pyrimidine-5-carbamate.
9. Propyl 7-[3,6-dihydro-1(2H)-pyridyl]-2-oxo-2H-[1,2,4] oxadiazolo [2,3-c] pyrimidine-5-carbamate.
10. Isopropyl 7-[3,6-dihydro-1(2H)-pyridyl]-2-oxo-2H-[1,2,4] oxadiazolo [2,3-c] pyrimidine-5-carbamate.
11. Allyl 7-[3,6-dihydro-1(2H)-pyridyl]-2-oxo-2H-[1,2,4] oxadiazolo [2,3-c] pyrimidine-5-carbamate.
12. Salts of the compounds in accordance with claims 5-11.
13. 5-Amino-7-[3,6-dihydro-1(2H)-pyridyl]-2H-[1,2,4]-oxadiazolo [2,3-c] pyrimidin-2-one of the formula

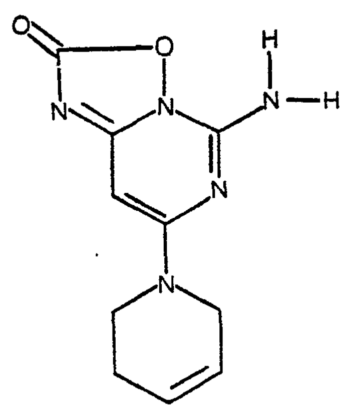

(II)

14. Oxadiazolopyrimidine derivatives in accordance with any one of claims 1-11 and their pharmaceutically usable salts as pharmaceutically active substances.
15. Oxadiazolopyrimidine derivatives in accordance with any one of claims 1-11 and their pharmaceutically usable salts as antihypertensives or vasodilators.
16. A process for the manufacture of oxadiazolopyrimidine derivatives of the general formula I defined in claim 1 and their salts, characterized by

a) reacting 5-amino-7-[3,6-dihydro-1(2H)-pyridyl]-2H-[1,2,4] oxadiazolo [2,3-c] pyrimidin-2-one of the formula

(See schema page 12)

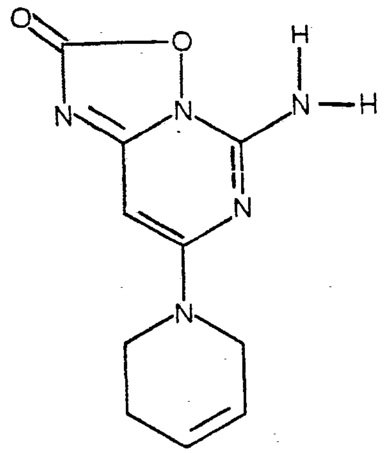

(II)

with an azole derivative of the general formula

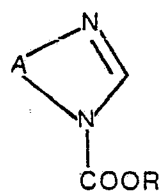

(III)

wherein R has the significance given in claim 1 and A signifies the groups —CH=CH—,

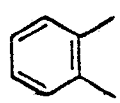

or —N=CH—, whereby the nitrogen atom in the latter case is situated in the 2-position, and

b) if desired, converting a compound of formula I obtained into a salt or converting a salt into a different salt.

17. A medicament containing an oxadiazolopyrimidine derivative in accordance with any one of claims 1-11 or a pharmaceutically usable salt thereof.

18. An antihypertensive or vasodilator containing an oxadiazolopyrimidine derivative in accordance with any one of claims 1-11 or a pharmaceutically usable salt thereof.

**Claims** (for the Contracting State AT)

1. A process for the manufacture of oxadiazolopyrimidine derivatives of the general formula

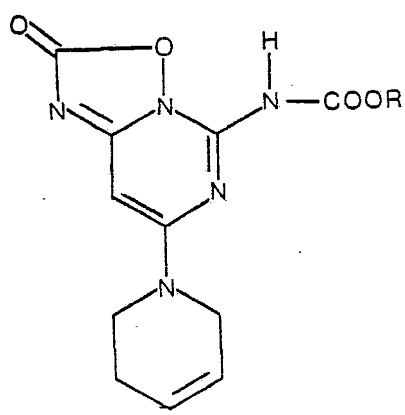

(I)

wherein R signifies $C_1$-$C_8$-alkyl, $C_3$-$C_6$-alkenyl, $C_3$-$C_6$-alkynyl or $C_2$-$C_8$-alkoxyalkyl, and their salts, characterized by

a) reacting 5-amino-7-[3,6-dihydro-1(2H)-pyridyl]-2H-[1,2,4] oxadiazolo [2,3-c] pyrimidin-2-one of the formula

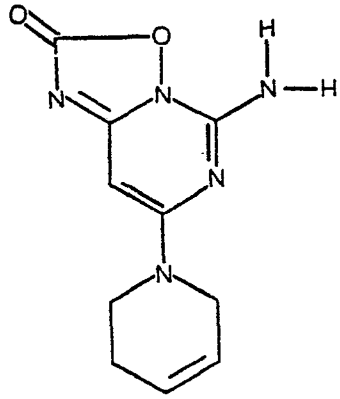

(II)

with an azole derivative of the general formula

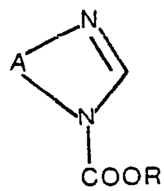

(III)

wherein R has the significance given above and A signifies the groups —CH=CH—,

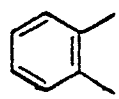

or —N=CH—, whereby the nitrogen atom in the latter case is situated in the 2-position, and

b) if desired, converting a compound of formula I obtained into a salt or converting a salt into a different salt.

2. A process according to claim 1, characterized in that R signifies $C_1$-$C_8$-alkyl or $C_2$-$C_8$-alkoxyalkyl.

3. A process according to claim 1, characterized in that R signifies $C_1$-$C_8$-alkyl or $C_3$-$C_6$-alkenyl.

4. A process according to claim 3, characterized in that R signifies alkyl with 1-4 carbon atoms or allyl.

5. A process according to claim 2, characterized in that methyl 7-[3,6-dihydro-1(2H)-pyridyl]-2-oxo-2H-[1,2,4] oxadiazolo [2,3-c] pyrimidine-5-carbamate is manufactured.

6. A process according to claim 2, characterized in that ethyl 7-[3,6-dihydro-1(2H)-pyridyl]-2-oxo-2H-[1,2,4] oxadiazolo [2,3-c] pyrimidine-5-carbamate is manufactured.

7. A process according to claim 2, characterized in that isobutyl 7-[3,6-dihydro-1(2H)-pyridyl]-2-oxo-2H-[1,2,4] oxadiazolo [2,3-c] pyrimidine-5-carbamate is manufactured.

8. A process according to claim 2, characterized in that butyl 7-[3,6-dihydro-1(2H)-pyridyl]-2-oxo-2H-[1,2,4] oxadiazolo [2,3-c] pyrimidine-5-carbamate is manufactured.

9. A process according to claim 4, characterized in that propyl 7-[3,6-dihydro-1(2H)-pyridyl]-2-oxo-2H-[1,2,4] oxadiazolo [2,3-c] pyrimidine-5-carbamate is manufactured.

10. A process according to claim 4, characterized in that isopropyl 7-[3,6-dihydro-1(2H)-pyridyl]-2-oxo-2H-[1,2,4] oxadiazolo [2,3-c] pyrimidine-5-carbamate is manufactured.

11. A process according to claim 4, characterized in that allyl 7-[3,6-dihydro-1(2H)-pyridyl]-2-oxo-2H-[1,2,4] oxadiazolo [2,3-c] pyrimidine-5-carbamate is manufactured.

12. A process according to claim 1, characterized in that a salt of one of the compounds in accordance with any one of claims 5-11 is manufactured.

**Revendications** (pour les Etats contractants : BE, CH, DE, FR, GB, IT, LI, LU, NL, SE)

1. Dérivés d'oxadiazolopyrimidine de formule générale

(Voir schéma page 14)

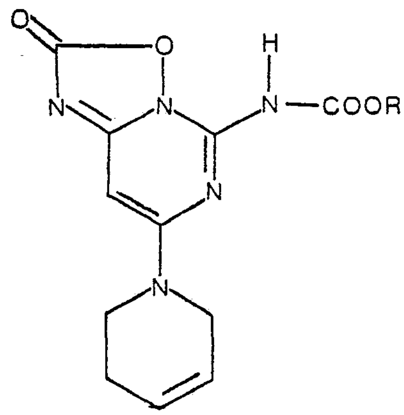

(I)

où R représente un alcoyle en $C_1$ à $C_8$, un alcényle en $C_3$ à $C_6$, un alcynyle en $C_3$ à $C_6$ ou un alcoxyalcoyle en $C_2$ à $C_8$, ainsi que leurs sels.

2. Composés selon la revendication 1, où R représente un alcoyle en $C_1$ à $C_8$ ou un alcoxyalcoyle en $C_2$ à $C_8$.

3. Composés selon la revendication 1, où R représente un alcoyle en $C_1$ à $C_8$ ou un alcényle en $C_3$ à $C_6$.

4. Composés selon la revendication 3, où R représente un alcoyle en $C_1$ à $C_4$ ou un allyle.

5. Méthyl 7-[3,6-dihydro-1(2H)-pyridyl]-2-oxo-2H-[1,2,4] oxadiazolo [2,3-c] pyrimidine-5-carbamate.

6. Ethyl 7-[3,6-dihydro-1(2H)-pyridyl]-2-oxo-2H-[1,2,4] oxadiazolo [2,3-c] pyrimidine-5-carbamate.

7. Isobutyl 7-[3,6-dihydro-1(2H)-pyridyl]-2-oxo-2H-[1,2,4] oxadiazolo [2,3-c] pyrimidine-5-carbamate.

8. Butyl 7-[3,6-dihydro-1(2H)-pyridyl]-2-oxo-2H-[1,2,4] oxadiazolo [2,3-c] pyrimidine-5-carbamate.

9. Propyl 7-[3,6-dihydro-1(2H)-pyridyl]-2-oxo-2H-[1,2,4] oxadiazolo [2,3-c] pyrimidine-5-carbamate.

10. Isopropyl 7-[3,6-dihydro-1(2H)-pyridyl]-2-oxo-2H-[1,2,4] oxadiazolo [2,3-c] pyrimidine-5-carbamate.

11. Allyl 7-[3,6-dihydro-1(2H)-pyridyl]-2-oxo-2H-[1,2,4] oxadiazolo [2,3-c] pyrimidine-5-carbamate.

12. Sels des composés selon les revendications 5-11.

13. 5-Amino-7-[3,6-dihydro-1(2H)-pyridyl]-2H-[1,2,4] oxadiazolo [2,3-c] pyrimidin-2-one de formule

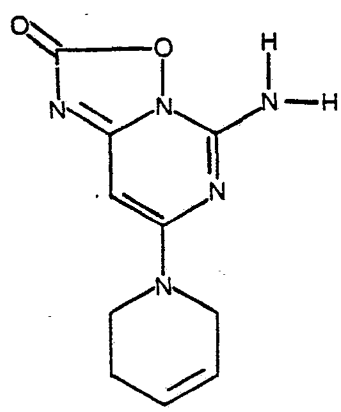

(II)

14. Dérivés d'oxadiazolopyrimidine selon l'une des revendications 1-11 et leurs sels pharmaceutiquement acceptables comme substances actives pharmaceutiques.

15. Dérivés d'oxadiazolopyrimidine selon l'une des revendications 1-11 et leurs sels pharmaceutiquement acceptables comme agents antihypertensifs ou vasodilatateurs.

16. Procédé de préparation de dérivés d'oxadiazolopyrimidine de formule générale I définie dans la revendication 1 et de leurs sels, caractérisé en ce que

a) on fait réagir la 5-amino-7-[3,6-dihydro-1(2H)-pyridyl]-2H-[1,2,4] oxadiazolo-[2,3-c] pyrimidin-2-one de formule

(Voir schéma page 15)

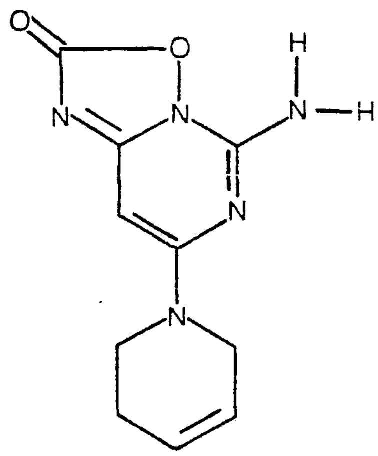

(II)

avec un dérivé azole de formule générale

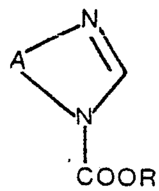

(III)

où R a la signification donnée dans la revendication 1 et A représente l'un des groupes —CH=CH—,

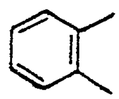

ou —N=CH— où l'atome d'azote dans ce dernier cas se trouve en position 2, et

b) si on le désire on transforme un composé de formule I obtenu en un sel ou un sel en un autre sel.

17. Médicament contenant un dérivé d'oxadiazolopyrimidine selon l'une des revendications 1-11 ou un de ses sels pharmaceutiquement acceptables.

18. Agent antihypertensif ou vasodilatateur contenant un dérivé d'oxadiazolopyrimidine selon l'une des revendications 1-11 ou un de ses dérivés pharmaceutiquement acceptables.

**Revendications** (pour l'Etat contractant AT)

1. Procédé de préparation de dérivés d'oxadiazolopyrimidine de formule générale

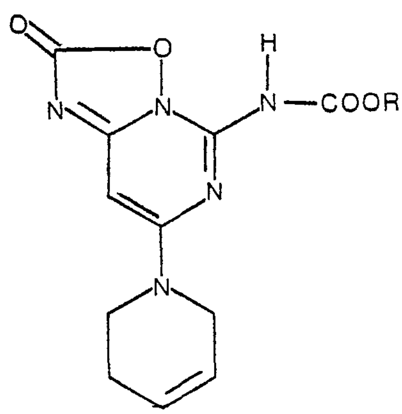

(I)

où R représente un alcoyle en $C_1$ à $C_8$, un alcényle en $C_3$ à $C_6$, un alcynyle en $C_3$ à $C_6$ ou un alcoxyalcoyle en $C_2$ à $C_8$, et de leurs sels, caractérisé en ce que

a) on fait réagir la 5-amino-7-[3,6-dihydro-1(2H)-pyridyl]-2H-[1,2,4] oxadiazolo-[2,3-c] pyrimidin-2-one de formule

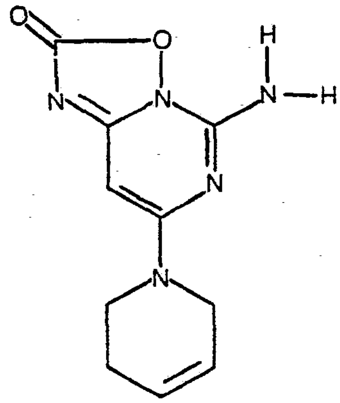

(II)

avec un dérivé azole de formule générale

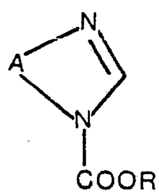

(III)

où R a la signification donnée ci-dessus et A représente l'un des groupes —CH=CH—,

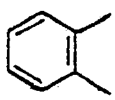

ou —N=CH— où l'atome d'azote dans ce dernier cas se trouve en position 2, et

b) si on le désire on transforme un composé obtenu de formule I en un sel ou un sel en un autre sel.

2. Procédé selon la revendication 1, caractérisé en ce que R représente un alcoyle en $C_1$ à $C_8$ ou un alcoxyalcoyle en $C_2$ à $C_8$.

3. Procédé selon la revendication 1, caractérisé en ce que R représente un alcoyle en $C_1$ à $C_8$ ou un alcényle en $C_3$ à $C_6$.

4. Procédé selon la revendication 3, caractérisé en ce que R représente un alcoyle en $C_1$ à $C_4$ ou un allyle.

5. Procédé selon la revendication 2, caractérisé en ce qu'on prépare le méthyl 7-[3,6-dihydro-1(2H)-pyridyl]-2-oxo-2H-[1,2,4] oxadiazolo [2,3-c] pyrimidine-5-carbamate.

6. Procédé selon la revendication 2, caractérisé en ce qu'on prépare l'éthyl 7-[3,6-dihydro-1(2H)-pyridyl]-2-oxo-2H-[1,2,4] oxadiazolo [2,3-c] pyrimidine-5-carbamate.

7. Procédé selon la revendication 2, caractérisé en ce qu'on prépare l'isobutyl 7-[3,6-dihydro-1(2H)-pyridyl]-2-oxo-2H-[1,2,4] oxadiazolo [2,3-c] pyrimidine-5-carbamate.

8. Procédé selon la revendication 2, caractérisé en ce qu'on prépare le butyl 7-[3,6-dihydro-1(2H)-pyridyl]-2-oxo-2H-[1,2,4] oxadiazolo [2,3-c] pyrimidine-5-carbamate.

9. Procédé selon la revendication 4, caractérisé en ce qu'on prépare le propyl 7-[3,6-dihydro-1(2H)-pyridyl]-2-oxo-2H-[1,2,4] oxadiazolo [2,3-c] pyrimidine-5 carbamate.

10. Procédé selon la revendication 4, caractérisé en ce qu'on prépare l'isopropyl 7-[3,6-dihydro-1(2H)-pyridyl]-2-oxo-2H-[1,2,4] oxadiazolo [2,3-c] pyrimidine-5-carbamate.

11. Procédé selon la revendication 4, caractérisé en ce qu'on prépare l'allyl 7-[3,6-dihydro-1(2H)-pyridyl]-2-oxo-2H-[1,2,4] oxadiazolo [2,3-c] pyrimidine-5-carbamate.

12. Procédé selon la revendication 1, caractérisé en ce qu'on prépare un sel d'un des composés selon l'une des revendications 5-11.